# FASCICULE DE BREVET EUROPEEN

(11) **EP 1 305 402 B1**
(45) Date de publication et mention de la délivrance du brevet: **29.03.2006**
(21) Numéro de dépôt: 01960853.8
(22) Date de dépôt: 31.07.2001
(51) Int. Cl.: C12N 7/02, G01N 33/576, A61K 39/29, C12Q 1/68, C12Q 1/70, A61P 31/14, A61K 35/18

(54) **COMPLEXE DE LIPO-VIRO-PARTICULES, PROCEDE DE PREPARATION ET APPLICATIONS**
KOMPLEX AUS DEN TEILEN DES HEPATITIS C VIRUS UND LIPOPROTEIN GERINGER DICHTE, VERFAHREN ZU SEINER HERSTELLUNG UND ANWENDUNGEN
LIPO-VIRO-PARTICLE COMPLEXES, PREPARATION METHOD AND USES

(30) Priorité: 31.07.2000 FR 0010085
(43) Date de publication de la demande: 02.05.2003
(73) Titulaire: BIO MERIEUX, 69280 Marcy l'Etoile (FR); INSTITUT NATIONAL DE LA SANTE ET DE LA RECHERCHE MEDICALE (INSERM), 75654 Paris Cédex 13 (FR)
(72) Inventeur: ANDRE, Patrice, F-69003 Lyon (FR); LOTTEAU, Vincent, F-69390 Vourles (FR); PARANHOS-BACCALA, Glaucia, F-69250 Lyon (FR); KOMURIAN-PRADEL, Florence, F-69250 Poleymieux (FR)
(74) Mandataire: Guerre, Dominique
(86) Numéro de dépôt international: PCT/FR2001/002507
(87) Numéro de publication internationale: WO 2002/010353

(56) Documents cités:
- WO-A-97/22349
- DE-C- 4 206 574
- US-A- 5 679 342
- US-A- 5 766 919
- THOMSSEN R ET AL: "DENSITY HETEROGENEITIES OF HEPATITIS C VIRUS IN HUMAN SERA DUE TO THE BINDING OF BETA-LIPOPROTEINS AND IMMUNOGLOBULINS" MEDICAL MICROBIOLOGY AND IMMUNOLOGY,BERLIN,DE, vol. 182, 1993, pages 329-334, XP000864518 cité dans la demande
- HIJIKATA M ET AL: "EQUILIBRIUM CENTRIFUGATION STUDIES OF HEPATITIS C VIRUS: EVIDENCE FOR CIRCULATING IMMUNE COMPLEXES" JOURNAL OF VIROLOGY,NEW YORK, US,US, vol. 67, no. 4, avril 1993 (1993-04), pages 1953-1958, XP000864525 ISSN: 0022-538X cité dans la demande
- SEIPP S ET AL: "ESTABLISHMENT OF PERISTENT HEPATITIS C VIRUS INFECTION AND REPLICATION IN VITRO" JOURNAL OF GENERAL VIROLOGY,SOCIETY FOR GENERAL MICROBIOLOGY, READING,GB, vol. 78, no. 10, 1997, pages 2467-2476, XP000960778 ISSN: 0022-1317
- ANDRE ET AL: J. VIROL., vol. 76, no. 14, juillet 2002 (2002-07), pages 6919-6928,

## Description

L'hépatite C est la cause principale des hépatites acquises par transfusion. L'hépatite C peut également être transmise par d'autres voies percutanées, par exemple par injection de drogues par voie intraveineuse. Le risque de contamination des professionnels de la santé n'est par ailleurs pas négligeable.

L'hépatite C se distingue des autres formes de maladies du foie associées à des virus, telles que les hépatites A, B ou D. Les infections par le virus de l'hépatite C (HCV ou VHC) sont souvent chroniques avec pour résultante des maladies du foie, telles que hépatite, cirrhose et carcinome dans un grand nombre de cas.

Bien que le risque de transmission du virus par transfusion ait diminué du fait de la sélection des donneurs de sang, la fréquence des hépatites C reste élevée. Actuellement, environ 170 millions de personnes à travers le monde sont infectées de manière chronique par HCV. Les populations à risque élevé se trouvent principalement chez les transfusés et les utilisateurs de drogues intraveineuses, mais il existe des donneurs de sang asymptomatiques qui n'appartiennent pas à ces groupes à risque élevé et chez lesquels des anticorps anti-HCV circulants ont été retrouvés. Pour ces derniers, la voie de l'infection n'a encore pas été identifiée.

HCV a été le premier virus hépatotrope isolé au moyen des techniques de biologie moléculaire. Les séquences du génome viral ont été clonées avant que la particule virale ait été visualisée.

HCV est un virus à ARN simple brin positif, de 9,5 kb, qui se réplique par une copie d'ARN complémentaire et dont le produit de la traduction est un précurseur d'une polyprotéine unique d'environ 3 000 acides aminés. L'extrémité 5' du génome de HCV correspond à une région non traduite adjacente aux gènes qui codent pour les protéines structurales, la protéine core de la nucléocapside et les deux glycoprotéines d'enveloppe, E1 et E2/NS1. La région non traduite 5' et le gène core sont relativement bien conservés dans les différents génotypes, mais les protéines d'enveloppe E2 sont codées par une région hypervariable différente d'un isolat à un autre isolat. L'extrémité 3' du génome de HCV contient les gènes qui codent pour les protéines non structurales (NS) et pour une région 3' non codante bien conservée.

Du fait de son organisation génomique et de son mode présumé de réplication, HCV a été classifié dans un nouveau genre de la famille des *Flaviviridae,* les *Hepacivirus.*

De nombreuses techniques ont été développées pour diagnostiquer une infection par HCV. Par exemple, des essais immunologiques de diagnostic ont été réalisés pour détecter des anticorps dirigés contre des protéines de HCV dans les sérums de patients. La synthèse d'ADNc par transcription inverse de l'ARN viral et l'amplification par PCR ont également été utilisées pour détecter le génome de HCV, comme la mesure indirecte d'un virus potentiellement infectieux dans les sérums d'humains infectés de manière chronique ou ceux de chimpanzés infectés expérimentalement. Par ailleurs, sur la base du clonage de gènes, des techniques d'hybridation avec une sonde ADN ont également été développées.

Cependant, il est reconnu que les techniques de diagnostic existantes manquent de sensibilité et/ou de spécificité et/ou souffrent de difficultés de mise en oeuvre. A titre d'exemple, avec la méthode d'hybridation de sondes il est impossible de faire la distinction entre un virus à faible pouvoir infectieux et un virus à pouvoir infectieux élevé. Il est donc nécessaire, mais difficile à mettre en oeuvre, d'inoculer le virus devant être testé à un chimpanzé et de tester l'infection résultante sur l'animal.

Il est donc de toute première importance, d'un point de vue de santé publique, de pouvoir développer des méthodes spécifiques, sensibles et pratiques pour identifier et cribler les porteurs de HCV. Une des solutions pourrait être de réaliser un système de culture in vitro très efficace de HCV qui permettrait d'obtenir une propagation du virus, en particulier pour étudier ses mécanismes de réplication, pour tester des anticorps neutralisants ou des antiviraux, de même que pour développer des matériaux biologiques, des essais de diagnostic et des préparations vaccinales. En effet, bien que la séquence complète de HCV soit disponible depuis 1989 (*Q. L Choo et al., Science* 244, 359 (1989)), la compréhension du cycle de vie et du mode de réplication d'HCV a été entravée par manque d'un système de culture in vitro approprié. Ito et al. (J. Gen. Virole 77 : 1043-1054 (1996)) ont bien confirmé le maintien de la réplication de HCV dans des cultures primaires d'hépatocytes humains, obtenus à partir de patients porteurs de HCV et pour lesquels la maladie était établie de manière chronique, et suggéré un passage d'infection, mais des problèmes relatifs à la propagation du virus subsistent (impossibilité de culture au long cours) et le système développé est limité par la nécessité d'approvisionnement en foie humain et la lourdeur de la technique. Par ailleurs, à ce jour, il n'y a pas de consensus général sur le tropisme de HCV et tous les récepteurs cellulaires pour le virus n'ont pas encore été identifiés.

Dans le plasma de patients infectés par HCV sont retrouvées des particules contenant de l'ARN viral très hétérogènes en densité. Cette hétérogénéité de densité des particules contenant de l'ARN viral est attribuée à leur association en proportion variable avec des lipoprotéines (Thomsen et al., 1993, Med. Microbiol. Immunol. 182 : 329-334). Dans la description de la présente demande de brevet, les inventeurs ont dénommé ces particules hybrides particules lipoprotéine - ARN viral, en abrégé LVPs (lipo-viro-particules). La repartition de chacune de ces formes le long d'un gradient de densité varie d'un patient à l'autre. Les analyses existantes des particules de faible densité montrent des densités recouvrant celles des LDLs (Low Density Lipoproteins) et des VLDLs (Very Low Density Lipoproteins). La taille décrite (50 nm) les rapprochent des VLDLs.

La nature des LVPs précitées contenant de l'ARN viral n'est à ce jour pas précisément connue, mais les présents inventeurs ont montré pour la première fois que les LVPs sont associées à des immunoglobumines humaines et que c'est dans ces fractions de LVPs, de densité égale ou inférieure à 1,063 g/ml, associées à des immunoglobulines humaines, qu'est retrouvé majoritairement l'ARN du virus HCV, contrairement aux données connues (Hijikata et al., J. Virol. (1993), 1953-1958). En effet, Hijikata et al. ont montré qu'il n'y avait pas d'immunoglobulines humaines dans les particules de densité inférieure à 1,06 g/ml, et que c'est dans les particules de cette densité qu'est retrouvée une forte infectivité chez le chimpanzé. Ces données expérimentales pourraient constituer au moins une des bases d'explication de la chronicité de la maladie, qui n'a pas encore été élucidée à ce jour et ouvrent de plus des perspectives pour de nouveaux procédés de culture in vitro du virus HCV.

Aussi, la présente invention a pour objet un complexe constitué de LVPs associées à des immunoglobulines humaines, ledit complexe ayant une densité inférieure à 1,063 g/ml et de préférence comprise entre 1,0063 et 1,063 g/ml, comme mis en évidence par centrifugation, par exemple sur gradient de bromure de sodium.

L'invention concerne aussi un procédé de préparation dudit complexe LVPs associées à des immunoglobulines humaines (LVP/Ig) qui consiste à réaliser une séparation par gradient de centrifugation à partir d'un prélèvement de plasma ou de sérum d'un patient pour l'obtention d'une fraction du complexe LVP/Ig de densité inférieure à 1,063 g/ml et de préférence comprise entre 1,0063 et 1,063 g/ml, puis une purification de la fraction dudit complexe par de la protéine A ou des anti-immunoglobulines humaines ou par tout autre molécule susceptible de lier les immunoglobulines humaines (couplées à un support, tel que des billes ou de la sepharose). Ce procédé permet « d'enrichir » le prélèvement de départ en complexe LVP/Ig en éliminant les lipoprotéines n'appartenant pas au complexe LVP/Ig.

Le complexe LVP/Ig obtenu, de préférence purifié selon le mode de préparation de l'invention est utilisable pour la mise au point d'un procédé de culture *in vitro* du virus HCV. En effet, un grand nombre de cellules possède à leur surface un récepteur du fragment Fc des immunoglobulines et il est ainsi possible de faire pénétrer l'ARN viral associé aux immunoglobulines dans ces cellules permissives, via l'interaction entre le fragment Fc des immunoglobulines et l'un de ses récepteurs membranaires, et d'assurer la propagation et la réplication du virus HCV *in vitro.*

Aussi, la présente invention a également pour objet un procédé de culture *in vitro* du virus HCV, selon lequel on met en contact, dans un milieu déterminé et sous des conditions appropriées, le complexe LVP/Ig (de préférence purifié selon le procédé de l'invention) et des cellules permissives qui comportent à leur surface au moins un type de récepteur du fragment Fc des immunoglobulines ou des cellules permissives exprimant au moins un récepteur pour une molécule ayant la capacité de lier les immunoglobulines, lesdites cellules permissives étant capables d'assurer la propagation et la réplication du virus HCV *in vitro.* Bien entendu l'expression de ce ou de ces récepteurs peut être soit spontanée soit induite, en particulier par transfection.

Parmi les cellules permissives exprimant au moins un type de récepteur du fragment Fc des immunoglobulines, on peut citer à titre d'exemples non limitatifs les cellules mononucléaires (cellules souches dérivées de la moelle osseuse, monoblastes, promonocytes, monocytes et macrophages, les cellules précurseurs des macrophages, les lymphocytes B, les cellules NK, les hépatocytes, les cellules denditriques, les cellules épithéliales, les cellules de l'endothélium vasculaire, les mastocytes, les cellules de Langerhans) ; les syncytiotrophoblastes, les polynucléaires éosinophiles, basophiles et neutrophiles, les plaquettes.

Parmi les cellules permissives exprimant au moins un récepteur pour une molécule ayant la capacité de lier des immunoglobulines, on peut citer les érythrocytes et leurs précurseurs, les macrophages, les monocytes, les leucocytes polymorphonucléaires (polynucléaires éosinophiles, basophiles et neutrophiles), les lymphocytes B et les cellules denditriques.

Les macrophages sont de préférence choisis dans le groupe qui consiste en histiocytes, macrophages alvéolaires, macrophages de la rate et du tissu lymphoïde, cellules de Kuppfer, osteoclastes, cellules synoviales de type A, macrophages tissulaires et les cellules précurseurs dont ces cellules sont dérivées. Parce que le virus HCV est hépatotrope, les cellules d'hépatocytes primaires humains ou animal, les cellules du groupe des lignées cellulaires d'hépatocarcinome humain ou animal et les cellules de Kuppfer sont avantageusement préférées. Mais comme il a été montré que HCV est capable de se propager et de se répliquer dans les lymphocytes, les lymphocytes B sont également des cellules permissives préférentielles.

Lorsque les cellules permissives sont des cellules d'hépatocytes primaires humains ou animal, des cellules du groupe des lignées cellulaires d'hépatocarcinome humain ou animal ou des cellules de Kuppfer, l'infection desdites cellules par le virus HCV est effectué via l'interaction entre le fragment Fc des immunoglobulines humaines et au moins un des récepteurs du fragment Fc présent à la surface desdites cellules permissives mais aussi via l'interaction des LVPs qui sont des ligands pour une voie d'endocytose associée aux récepteurs des lipoprotéines, tels que le LDL-récepteur et le LSR présents à la surface de ces mêmes cellules permissives.

Le terme virus HCV fait référence à toute espèce virale, parmi lesquelles les souches pathogènes pour l'homme, les souches atténuées et les souches défectives dérivées desdites souches. En effet, il est connu que les virus à ARN présentent un taux de mutations spontanées élevé. Il peut donc exister des souches multiples qui peuvent être plus ou moins virulentes. Il est à la portée de l'homme de l'art d'identifier de telles souches, par exemple par homologie de séquences nucléiques et/ou peptidiques par rapport à une souche de référence et/ou en identifiant une souche ou un isolat par rapport à des critères morphologiques et/ou immunologiques.

Le terme système cellulaire « *in vitro »* fait référence à des cellules qui ont été répliquées *in vitro* et inclut donc les cultures primaires, les cultures dérivées des cultures primaires, les lignées primaires, et les lignées dérivées desdites lignées primaires. Parce qu'il est connu que des modifications induites ou spontanées peuvent survenir dans le caryotype durant le stockage ou le transfert, les cellules dérivées d'une lignée cellulaire de référence peuvent ne pas être strictement identiques aux cellules ou cultures d'origine et l'invention inclut de tels variants.

Le terme lignée cellulaire fait référence aux lignées établies, immortalisées et spontanées. En pratique, pour effectuer une culture virale d'intérêt, il est nécessaire que le virus puisse se propager *in vitro* dans une culture dans laquelle les cellules sont capables de se multiplier en permanence et ainsi permettre la propagation virale. La lignée cellulaire est donc de préférence une lignée cellulaire établie ou qui résulte d'une immortalisation par différents procédés. Ceci peut être effectué (i) par l'établissement d'une lignée stable, établie, continue, par mise en co-culture de cellules permissives avec des cellules permissives de même nature tumorisées, capables de se multiplier indéfiniment et d'assurer la propagation du virus au sein de la culture, l'inoculation virale ayant lieu au sein de la culture, (ii) en utilisant des cellules primaires infectées par le virus qui sont ensuite co-cultivées avec des cellules tumorisées permissives qui assurent la propagation du virus au sein de la culture de la lignée cellulaire ainsi établie ou encore (iii) par infection virale d'une lignée cellulaire, par exemple une lignée de lymphocytes B immortalisée, par exemple par le virus d'Epstein-Barr.

Le milieu approprié retenu pour la culture de HCV est le milieu RPMI ou un milieu dérivé du milieu RPMI. De préférence, c'est le milieu RPMI 1640 supplémenté en :
pénicilline 1%
streptomycine 1%
glutamine 2 mM.
SVF (sérum de veau foetal) décomplémenté 10%.

Eventuellement pour la culture de certaines cellules permissives le milieu précité comprend de plus du beta-mercaptoéthanol 50 *µ*M.

On réalise plusieurs passages des cellules permissives ainsi infectées et on met en évidence la présence dudit virus dans les cellules permissives infectées et dans le surnageant de culture par RT-PCR et/ou par une technique immunologique, telle que immunofluorescence indirecte à l'aide d'un anticorps spécifique dudit virus et/ou par cytométrie de flux.

Le procédé de l'invention, qui permet d'obtenir une propagation du virus HCV, est utile notamment pour étudier son mécanisme de réplication, pour tester des anticorps neutralisants et des antiviraux, pour développer des matériaux biologiques pour le diagnostic et la thérapie. Par ailleurs, le procédé de l'invention permet d'obtenir une lignée cellulaire infectée utile pour cribler et/ou sélectionner au moins une molécule anti-virale, par mise en contact de la lignée cellulaire infectée et de la molécule anti-virale.

L'invention concerne encore un procédé pour préparer une composition pour la détection dans un échantillon d'anticorps dirigés contre HCV qui comprend au moins une purification partielle ou totale des particules virales dudit virus ou des polypeptides obtenus à partir du procédé de l'invention. Par purification partielle ou total, on entend par exemple une purification par ultracentrifugation sur gradient de sucrose, par précipitation différentielle en sulfate d'ammonium, une chromatographie sur gel ou tout autre procédé bien connu de l'homme du métier. En particulier, lesdites particules virales ou lesdits polypeptides sont fixés sur un support solide.

Par ailleurs, l'invention concerne un procédé pour l'obtention d'anticorps ou de fragments d'anticorps dirigés contre le virus HCV selon lequel on immunise un animal avec le complexe constitué de LVPs associées à des immunoglobulines humaines ayant une densité inférieure à 1,063 g/ml, de préférence comprise entre 1,0063 et 1,063 g/ml ou avec des fraction dudit complexe ; ledit complexe étant éventuellement préparé selon le procédé décrit ci-dessus. Le complexe peut ainsi être soumis à une étape de traitement préalable à l'immunisation, par exemple par traitement à des détergents ou à des agents chaotropiques, pour l'obtention de fractions constituées de protéines virales, de lipides et de phospholipides. La production d'anticorps polyclonaux et monoclonaux ou de fragments d'anticorps fait partie des connaissances générales de l'homme du métier. On peut citer à titre d'exemple Kôhler G. et Milstein C. (1975) Continuous culture of fused cells secreting antibody of predefined specificity, Nature, 256 : 495-497 et Galfre G. et al. (1977) Nature, 266 : 522-550 pour la production d'anticorps polyclonaux et Roda A., Bolelli G.F. Production of high titer antibody to bile acids, Journal os Steroid Biochemistry, Vol. 13, pp 449-454 (1980) pour la production d'anticorps polyclonaux. Les anticorps sont produits par immunisation de souris ou de lapins avec le complexe LVP/Ig+ ou avec-des fractions dudit complexe comprenant des protéines virales, des lipides et des phospholipides. Les animaux sont soumis à une injection d'immunogène en utilisant de l'adjuvant complet de Freund. Les sérums et les surnageants de culture d'hybridome issus des animaux immunisés sont analysés pour leur spécificité et leur sélectivité en utilisant des techniques classiques, telles que par exemple des tests ELISA ou de Western Blot. Les hybridomes produisant les anticorps les plus spécifiques et les plus sensibles sont sélectionnés. Des anticorps monoclonaux peuvent également être produits *in vitro* par culture cellulaire des hybridomes produits ou par récupération de liquide d'ascite, après injection intrapéritonéale des hybridomes chez la souris. Quelque soit le mode de production, en surnageant ou en ascite, les anticorps sont ensuite purifiés. Les méthodes de purification utilisées sont essentiellement la filtration sur gel échangeur d'ions et la chromatographie d'exclusion ou l'immunoprécipitation. Un nombre suffisant d'anticorps sont criblés dans des tests fonctionnels pour identifier les anticorps les plus performants. La production in vitro d'anticorps, de fragments d'anticorps ou de dérivés d'anticorps, tels que des anticorps chimères produits par génie génétique est bien connue de l'homme du métier.

Plus particulièrement, par fragment d'anticorps on entend les fragments F(ab)2, Fab, Fab', sFv (Blazar et al., 1997, Journal of Immunology 159 : 5821-5833 et Bird et al., 1988, Science 242 : 423-426) d'un anticorps natif et par dérivé on entend, entre autres, un dérivé chimérique d'un anticorps natif (voir par exemple Arakawa et al., 1996, J. Biochem 120 : 657-662 et Chaudray et al., 1989, Nature 339 : 394-397).

L'invention concerne également un procédé de culture in vitro du virus HCV selon lequel on dispose d'un complexe constitué de LVPs associées à des immunoglobulines humaines (LVP/Ig) tel que défini précédemment, on met en contact ledit complexe avec de la protéine A pour l'obtention d'un complexe LVP/Ig/protéine A, on met en contact ledit complexe LVP/Ig/protéine A avec au moins un anticorps spécifique d'au moins un récepteur cellulaire pour l'obtention d'un complexe LVP/Ig/protéine A/anticorps et on met en contact, dans un milieu de culture et sous des conditions appropriées, ledit complexe LVP/Ig/protéine A/anticorps et des cellules permissives qui comportent ou expriment à leur surface au moins un récepteur cellulaire ayant la capacité de lier l'anticorps ; lesdites cellules permissives étant capables d'assurer la propagation et la réplication du virus HCV in vitro. Selon ce procédé et de préférence :
- la protéine A est couplée à un support, tel que des billes ou de la sepharose,
- l'anticorps est anticorps spécifique d'au moins un des récepteurs des LDLs et les cellules permissives comportent ou expriment à leur surface au moins un récepteur des LDLs,
- les cellules permissives sont choisies parmi les hépatocytes primaires humains ou animaux et les cellules du groupe des lignées cellulaires d'hépatocarcinome humain ou animal, telles que les cellules de la lignée d'hépatocarcinome humain HepG2,
- le milieu de culture est le milieu DMEM supplémenté en SVF 10%.
- on met en évidence la présence du virus HCV dans les cellules permissives par RT-PCR et/ou par technique immunologique, telle que par immunofluorescence indirecte, notamment à l'aide d'un anticorps spécifique dudit virus et/ou par cytométrie de flux.

Dans le procédé de l'invention la protéine A est ajoutée en excès, ce qui signifie que de la protéine A qui ne s'est pas liée aux immunoglobulines humaines des LVP/Ig est encore disponible, adsorbée à la surface des LVP, pour se lier à l'anticorps spécifique d'un récepteur cellulaire déterminé. Bien entendu, l'anticorps peut être un anticorps polyclonal ou monoclonal, mais de préférence sera un anticorps monoclonal pour assurer une meilleure spécificité.

L'invention concerne encore un procédé pour préparer une composition pour la détection dans un échantillon d'anticorps dirigés contre le virus HCV qui comprend au moins une purification partielle ou totale des particules virales de HCV ou des polypeptides obtenus à partir d'un procédé de culture tel que défini ci-dessus. De préférence pour la préparation de ladite composition les particules virales ou les polypeptides sont fixés sur un support solide.

L'invention a aussi pour objet un procédé pour le chargement in vitro de cellules présentatrices d'antigènes (APCs), selon lequel on dispose d'un complexe constitué de LVPs associées à des immunoglobulines humaines (LVP/Ig) tel que défini dans l'une quelconque des revendications 1 et 2, on met en contact ledit complexe avec de la protéine A pour l'obtention d'un complexe LVP/Ig/protéine A, on met en contact ledit complexe LVP/Ig/protéine A avec au moins un anticorps spécifique d'au moins un récepteur cellulaire des cellules présentatrices d'antigènes (APCs) prélevées chez un être humain ou animal pour l'obtention d'un complexe LVP/Ig/protéine A/anticorps et on met en contact ledit complexe LVP/Ig/protéine A/anticorps spécifique avec lesdites cellules présentatrices d'antigènes. Les APCs interviennent dans le processus d'apprêtement des antigènes exogènes pour produire des peptides immunogènes qui se lient aux molécules du Complexe Majeur d'Histocompatibilité de classe I ou II permettant la stimulation des lymphocytes. Le terme APCs recouvre généralement les macrophages, les cellules B et les cellules dendritiques. Dans la présente invention, on s'est particulièrement focalisé sur les cellules dendritiques, mais ceci n'est nullement limitatif et le procédé englobe tout anticorps qui est capable de se lier à au moins un récepteur des APCs. Quand les cellules présentatrices d'antigènes sont les cellules dendritiques, l'anticorps est un anticorps spécifique d'au moins un récepteur cellulaire des cellules dendritiques choisi parmi les « scavenger » récepteur A et B, le récepteur du mannose et les « Toll Like receptors » (TLRs). Dans le procédé de chargement de l'invention les APCs sont autologues, ce qui signifie qu'elles sont prélevées chez un être humain ou animal et qu'après chargement *in vitro* selon le procédé de l'invention elles seront réintroduites chez le même être humain ou animal pour induire une réponse humorale et cellulaire. On parle généralement de thérapie cellulaire.

L'invention concerne encore un procédé pour l'obtention d'anticorps ou de fragments d'anticorps dirigés contre le virus HCV, selon lequel on immunise un animal avec un complexe LVP/Ig/protéine A/anticorps susceptible d'être obtenu par un procédé selon lequel on dispose d'un complexe constitué de LVPs associées à des immunoglobulines humaines (LVP/Ig) tel que défini précédemment, on met en contact ledit complexe avec de la protéine A pour l'obtention d'un complexe LVP/Ig/protéine A, on met en contact ledit complexe LVP/Ig/protéine A avec au moins un anticorps spécifique d'au moins un récepteur cellulaire de cellules présentatrices d'antigènes (APCs) pour l'obtention d'un complexe LVP/Ig/protéine A/anticorps. De préférence, les cellules présentatrices d'antigènes sont les cellules dendritiques et l'anticorps est un anticorps spécifique d'au moins un récepteur cellulaire des cellules dendritiques choisi parmi les « scavenger » récepteur A et B, le récepteur du mannose et les « Toll Like receptors » (TLRs).
La figure 1 représente la quantité de LVP/Ig+ internalisée déterminée par quantification de l'ARN viral. En abscisse sont représentées les quantités en µg/ml d'anticorps anti-LDL récepteur et en ordonnée est représenté le nombre de copies d'ARN par µg de protéine A.
La figure 2 représente le nombre de copies d'ARN viral obtenu par puits en présence d'immunoglobulines humaines, d'immunoglobulines humaines et d'anticorps anti-LDL récepteur, d'anticorps anti-LDL récepteur et le contrôle.

### Exemple 1 : Préparation du matériel biologique.

La séparation des lipoprotéines est effectuée à partir du plasma ou du sérum d'un patient à jeun depuis 12 heures et détecté positif pour le virus de l'hépatite C.

Au sang prélevé du patient est ajouté 1% d'une solution d'EDTA (0,15 M NaCl - 0,1 M EDTA). Le mélange est centrifugé pendant 10 minutes à 3500 tpm, à la température de 4°C. Le plasma est ensuite récolté et stocké à 4°C jusqu'à utilisation.

Le sang du patient est récolté sur tube sec et, après coagulation, centrifugé pendant 10 minutes à 3000 tpm, à la température de 4°C. Le sérum est prélevé et stocké à 4°C jusqu'à utilisation.
(i) Obtention d'une fraction comprenant des LVPs d'une densité inférieure à 1,0063 g/ml, d'une fraction comprenant des LVPs d'une densité comprise entre 1,0063 g/ml et 1,063 g/ml, et d'une fraction comprenant des LVPs supérieure à 1,063 g/ml.
   Le plasma et le sérum sont respectivement ultracentrifugés pendant 4 heures à 100 000 tpm, à 4°C dans un appareil TL100 commercialisé par la société Beckman et comprenant un rotor TL100.4. La fraction supérieure qui contient les LVPs de densité inférieure à 1,0063 g/ml est récupérée et conservée à 4°C. La fraction inférieure est ajustée à une densité de 1,063 g/ml par addition de 7,21 g de NaBr pour 100 ml de la fraction. La fraction inférieure est ensuite ultracentrifugée pendant 4 heures à 100 000 tpm, à 4°C. dans un appareil TL100 comprenant un rotor TL100.4. La fraction supérieure résultante contenant la fraction d'une densité comprise entre 1,0063 g/ml et 1,063 g/ml est récupérée et conservée à 4°C.
(ii) Obtention d'une fraction comprenant des LVP d'une densité inférieure à 1,025 g/ml, d'une fraction comprenant des LVPs d'une densité comprise entre 1,025 g/ml et 1,063 g/ml, et d'une fraction comprenant des LVP d'une densité supérieure à 1,063 g/ml.

Le plasma et le sérum sont respectivement ajustés à une densité finale de 1,025 g/ml par addition de 2,518 g de NaBr pour 100 ml. Une centrifugation est effectuée pendant 4 heures à 100 000 tpm, à 4°C sur l'appareil TL100 comprenant un rotor TL100.4.

La fraction supérieure qui contient la fraction d'une densité inférieure à 1,025 g/ml est récupérée et conservée à 4°C. La fraction inférieure est ajustée à une densité de 1,063 g/ml par addition de 4,84 g de NaBr pour 100 ml. La fraction inférieure est ensuite ultracentrifugée pendant 4 heures à 100 000 tpm, à 4°C. dans l'appareil TL100 comprenant un rotor TL100.4. La fraction supérieure résultante contenant les LDLs est récupérée et conservée à 4°C.

Les différentes fractions récoltées sont ensuite dialysées pendant 18 heures à 4°C contre le tampon 0,15 M NaCl/0,24 mM EDTA. Les fractions sont ensuite récupérées et filtrées sur une membrane de 0,45 *µ*. Un dosage de protéines est effectué par la méthode de Lowry (Sigma).

### Exemple 2 : Mise en évidence de l'association des LVPs à des immunoglobulines humaines.

Différentes fractions contenant des LVPs récoltées à partir de plasma de trois patients infectés par HCV et préparées selon le protocole de l'exemple 1 ont été analysées par électrophorèse sur gel de polyacrylamide (PAGE) en présence de SDS (Dodecyl Sulfate de Sodium) (SDS-PAGE) (Laemmli, Nature (1970), 227 :680-685). La mise en évidence de la présence d'immunoglobulines (Ig) dans ces fractions a été réalisée par la technique de Western blot (Towbin et al., PNAS, (1979) 76 : 4350-4354), à l'aide d'un sérum de chèvre anti-immunoglobulines humaines couplé à la peroxydase (Jackson ImmunoResearch laboratories, France). Les résultats montrent que les immunoglobulines humaines sont toujours détectées dans les fractions contenant des LVPs, en quantité différente selon les patients.

La quantité du génome HCV dans ces fractions contenant des LVPs a été mesurée par quantification de l'ARN de HCV par RT-PCR (RT = transcriptase inverse; PCR = polymerase chain reaction)et détection de fluorescence en temps réel (LIGHTCYCLER™, ROCHE) (Wittwer et al., Biotechniques (1997), 22 :176-181). Les résultats montrent que l'ARN de HCV est toujours associé aux fractions contenant des LVPs, en quantité différente selon les patients.

Les LVPs associées aux Ig (LVP/Ig+) ont de plus été purifiées à l'aide de la protéine A couplé à des billes de type MAGmol Protein A MicroBeads Miltenyi Biotec, France) après un passage dans des colonnes MS+ Separation Columns (Miltenyi Biotec, France)ou bien à l'aide de la Protéine A-Sepharose CL-4B (Pharmacia Biotech, France). Dans ce cas, tout ou majorité de l'ARN-HCV co-purifie avec les Ig, comme illustré dans les tableaux qui suivent. En conséquence, à partir d'une fraction riche en LVPs, les échantillons utilisés pour les infections peuvent être purifiés par l'intermédiaire de leurs Ig de manière à utiliser préférentiellement les LVP/Ig+/ARN+.

### Patient n°1

| | d* < 1,0063 | d* 1,063 <d> 1,0063 |
|---|---|---|
| Présence d'Ig | +++ | +++ |
| Quantification d'ARN (pour 0,2ml de LVPs) | 27300 copies | 33600 copies |
| Quantification d'ARN co-purifiés avec Ig | 23625 copies (86,5%) | 31875 copies (94,8%) |

| | | |
|---|---|---|
| d* signifie la densité des LVPs en g/ml. | | |

### Patient n°2

| | d* < 1,0063 | d* 1,063 <d> 1,0063 |
|---|---|---|
| Présence d'Ig | +++ | +/- |
| Quantification d'ARN (pour 0,2ml de LVP) | 32400 copies | 235800 copies |
| Quantification d'ARN co-purifiés avec Ig | 21300 copies (65,7%) | 21300 copies (9%) |

| | | |
|---|---|---|
| d* signifie la densité des LVPs en g/ml. | | |

### Patient n°3

| | d* < 1,0063 | d* 1,025 <d> 1,055 |
|---|---|---|
| Présence d'Ig | +++ | + |
| Quantification d'ARN (pour 0,2ml de LVP) | 197100 copies | 45900 copies |
| Quantification d'ARN co-purifiés avec Ig | 142500 copies (72,3%) | 26100 copies (56,8%) |

| | | |
|---|---|---|
| d* signifie la densité des LVPs en g/ml. | | |

Ces résultats montrent que lorsque des immunoglobulines sont présentes dans les fractions contenant des LVPs les ARNs viraux sont retrouvés majoritairement dans les fractions de LVPs associées à des immunoglobulines humaines.

### Exemple 3 : Internalisation des LVPs par ciblage cellulaire.

Lors de l'étape initiale de l'infection virale in vitro par le virus VHC purifié selon l'exemple 2, l'utilisation d'un anticorps dirigé contre un récepteur cellulaire permet d'augmenter l'efficacité du ciblage et de l'internalisation des LVPs dans une lignée cellulaire donnée.

Les LVPs associées aux Ig (LVP/Ig+) ont été purifiées à partir d'une fraction de densité comprise entre 1,025 et 1,055 g/ml à l'aide de la protéine A couplée aux billes de type MAGmol Protein A MicroBeads (Miltenyi Biotec, France) comme décrit dan l'exemple 2.

La lignée d'hépatocarcinome humain HepG2 cultivée en milieu DMEM-10% SFV a été utilisée pour l'étude de l'internalisation des LVP/Ig+ purifiées en présence d'un anticorps anti-LDL récepteur. Les résultats ont été obtenus selon le protocole suivant :

Les cellules HepG ont été ensemencées à 45000 cellules / puits sur une plaque 96 puits, de façon à obtenir après 24 heures d'incubation à 37°C - 5% CO₂, une confluence d'environ 70%.

Trois lavages en tampon PBS 1X ont été réalisés puis la fraction LVP/Ig+ (soit 0,25 x 10⁶ copies d'ARN VHC) a été rajoutée dans chaque puits en présence d'une quantité croissante d'anticorps anti-LDL récepteur : soit 0, 1, 5, 10 et 20 µg / ml. Quatre puits ont été réalisés par essai. L'incubation a été effectuée à +37°C pendant 3 heures.

Les cellules ont été lavées 3 fois à l'aide de PBS 1X / 0,2% BSA froid, puis traitées à la suramine 10 mM pendant 1 heure sur un lit de glace. Après trois nouveaux lavages en PBS1X, les cellules ont été lysées avec 350 *µ*l de tampon de lyse du kit Rneasy (Qiagen). Les ARN ont été ensuite purifiés à l'aide de ce même kit et analysés par RT-PCR quantitative (LightCycler™).

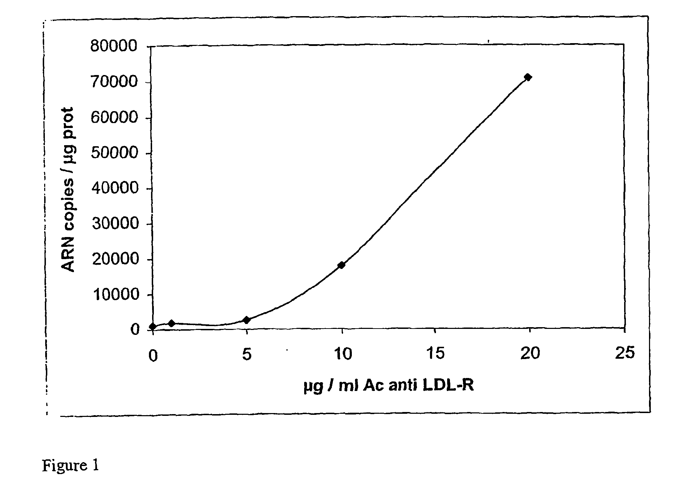

Les résultats obtenus sont indiqués dans la figure 1. Les résultats montrent que la quantité de LVP/Ig+ internalisée (déterminée par quantification de l'ARN viral) est d'autant plus importante que la quantité d'anticorps anti-LDL récepteur est élevée. Cela s'explique par la formation d'un complexe LVP/Ig+ - protéines A / bille magnétique - anticorps anti-LDL récepteur, qui se réalise grâce à la présence de protéines A libres et non couplées aux LVPs sur les billes magnétiques. Cette hypothèse est vérifiée dans l'expérience décrite ci-dessous.

Les LVPs associées aux Ig (LVP/Ig+) ont été purifiées à partir d'une fraction de densité inférieure à 1,006 g/ml à l'aide de la protéine A couplée aux billes de type MAGmol Protein A MicroBeads (Miltenyi Biotec, France) comme décrit dans l'exemple 2.

Les cellules HepG2 ont été ensemencées à 50000 cellules / puits (plaque 96 puit), de façon à obtenir après 24h00 d'incubation à 37°C - 5% CO₂, une confluence d'environ 80%. Les résultats ont été obtenus avec le protocole décrit précédemment, excepté que la fraction LVP/Ig+ (soit 0,4 x 10⁶ copies d'ARN VHC) a été rajoutée pour chaque essai avec différentes conditions :
- en présence de 50 *µ*g d'immunoglobulines humaines,
- en présence de 50 *µ*g d'immunoglobulines humaines et de 10 *µ*g / ml d'anticorps anti LDL récepteur,
- en présence de 10 *µ*g / ml d'anticorps anti LDL récepteur,
une pré-incubation des LVP/Ig+ avec les immunoglobulines humaines ayant été réalisée pendant 1 heure à température ambiante.

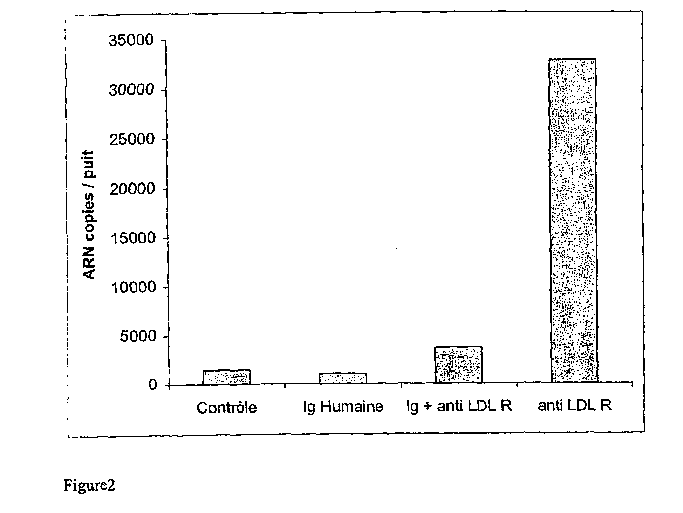

Les résultats obtenus sont indiqués dans la figure 2. Ces résultats confirment la spécificité du ciblage cellulaire, en ce sens que l'incubation des LVPs avec des immunoglobulines non spécifiques du récepteur bloquent la facilitation de l'internalisation par les anticorps anti-LDL récepteur.

## Revendications

1. Complexe constitué de lipo-viro-particules (LVPs) associées à des immunoglobulines humaines ayant une densité inférieure à 1,063 g/ml, susceptible d'être obtenu par le procédé selon lequel :
on dispose d'un prélèvement d'un échantillon de plasma ou de sérum d'un patient infecté par le virus de l'hépatite C (HCV),
on sépare dudit prélèvement les LVPs par centrifugation en fonction de leur densité, et
on sépare les LVPs associées à des immunoglobulines humaines à l'aide de la protéine A, d'anti-immunoglobulines humaines ou de tout autre molécule susceptible de lier les immunoglobulines humaines.

2. Complexe selon la revendication 1, ayant une densité comprise entre 1,0063 et 1,063 g/ml.

3. Procédé de préparation d'un complexe constitué de LVPs associées à des immunoglobulines humaines et ayant une densité inférieure à 1,063 g/ml, de préférence comprise entre 1, 0063 et 1, 063 g/ml, selon lequel :
on dispose d'un prélèvement d'un échantillon de plasma ou de sérum d'un patient infecté par HCV,
on sépare dudit prélèvement les LVPs par Centrifugation en fonction de leur densité, et
on sépare les LVPs associées à des immunoglobulines humaines à l'aide de la protéine A, d'anti-immunoglobulines humaines ou de tout autre molécule susceptible de lier les immunoglobulines humaines.

4. Procédé selon la revendication 3, dans lequel la protéine A, les anti-immunoglobulines humaines ou autre molécule susceptible de lier les immunoglobulines humaines sont couplées à un support, tel que des billes ou de la sepharose.

5. Procédé de culture *in vitro* du virus HCV selon lequel, on met en contact dans un milieu de culture et sous des conditions appropriées un complexe constitué de LVPs associées à des immunoglobulines humaines selon les revendications 1 et 2, éventuellement préparé selon les revendications 3 et 4 et des cellules permissives qui comportent à leur surface au moins un type de récepteur du fragment Fc des immunoglobulines, lesdites cellules permissives étant capables d'assurer la propagation et la réplication du virus HCV *in vitro.*

6. Procédé selon la revendication 5, selon lequel les cellules permissives sont choisies dans le groupe consistant en les cellules mononucléaires, telles que les cellules souches dérivées de la moelle osseuse, monoblastes, promonocytes, monocytes et macrophages, les cellules précurseurs des macrophages, les lymphocytes B, les cellules NK, les hépatocytes primaires humains ou animaux, les cellules du groupe des lignées cellulaires d'hépatocarcinome humain ou animal, les cellules de Kuppfer, les cellules denditriques, les cellules épithéliales, les cellules de l'endothélium vasculaire, les mastocytes, les cellules de Langerhans ; les syncytiotrophoblastes, les polynucléaires éosinophiles, basophiles et neutrophiles et les plaquettes.

7. Procédé selon la revendication 6 dans lequel les macrophages sont de préférence choisis dans le groupe qui consiste en histiocytes, macrophages alvéolaires, macrophages de la rate et du tissu lympholde, cellules de Kuppfer, osteoclastes, cellules synoviales de type A, macrophages tissulaires et les cellules précurseurs dont ces cellules sont dérivées.

8. Procédé selon la revendication 6, dans lequel les cellules permissives sont les hépatocytes primaires ou animaux, les cellules du groupe des lignées cellulaires d'hépatocarcinome humain ou animal ou les cellules de Kuppfer qui possèdent au moins un type de récepteur du fragment Fc des immunoglobulines et/ou au moins un récepteur des lipoprotéines tel que le LDL-récepteur et le récepteur Lypolysis Stimulated Receptor.

9. procédé selon l'une quelconque des revendications 5 à 8, dans lequel le milieu de culture est le milieu RPMI 1640 supplémenté en pénicilline 1%, streptomycine 1%, glutamine 2 mM, SVF 10%, et comprenant de plus éventuellement du beta-mercaptoéthanol 50 µM.

10. Procédé selon l'une quelconque des revendications 6 à 9, selon lequel on met en évidence la présence du virus HCV dans les cellules permissives par RT-PCR et/ou par technique immunologique, telle que par immunofluorescence indirecte, notamment à l'aide d'un anticorps spécifique dudit virus et/ou par cytométrie de flux.

11. Procédé pour préparer une composition pour la détection dans un échantillon d'anticorps dirigés contre le virus HCV, procédé selon lequel :
on met en contact dans un milieu de culture et sous des conditions appropriées un complexe constitué de LVPs associées à des immunoglobulines humaines selon les revendications 1 et 2, éventuellement préparé selon les revendications 3 et 4 et des cellules permissives qui comportent à leur surface au moins un type de récepteur du fragment Fc des immunoglobulines, lesdites cellules permissives étant capables d'assurer la propagation et la réplication du virus HCV *in vitro,* pour obtenir des particules virales de *HCV* ou des polypeptides, et
on purifie partiellement ou totalement lesdites particules virales de HCV ou lesdits polypeptides.

12. Procédé selon la revendication 11, dans lequel lesdites particules virales ou lesdits polypeptides sont fixés sur un support solide.

13. Procédé pour l'obtention d'anticorps ou de fragments d'anticorps dirigés contre le virus HCV, selon lequel on immunise un animal non-humain avec un complexe selon la revendication 1 ou 2, éventuellement préparé par un procédé selon la revendication 3 ou 4.

14. Procédé de culture in vitro du virus HCV selon lequel on dispose d'un complexe constitué de LVPs associées à des immunoglobulines humaines (LVP/Ig) tel que défini dans l'une quelconque des revendications 1 et 2, on met en contact ledit complexe avec de la protéine A pour l'obtention d'un complexe LVP/Tg/protéine A, on met en contact ledit complexe LVP/Ig/protéine A avec au moins un anticorps spécifique d'au moins un récepteur cellulaire pour l'obtention d'un complexe LVp/Ig/protéine A/anticorps et on met en contact, dans un milieu de culture et sous des conditions appropriées, ledit complexe LVP/Ig/protéine A/anticorps et des cellules permissives qui comportent ou expriment à leur surface au moins un récepteur cellulaire ayant la capacité de lier l'anticorps ; lesdites cellules permissives étant capables d'assurer la propagation et la réplication du virus HCV *in vitro*.

15. Procédé selon la revendication 14, dans lequel la protéine A est couplée à un support, tel que des billes ou de la sepharose.

16. Procédé selon la revendication 14 ou 15, dans lequel l'anticorps est anticorps spécifique d'au moins un des récepteurs des LDLs et les cellules permissives comportent ou expriment à leur surface au moins un récepteur des LDLs.

17. Procédé selon la revendication 16, dans lequel les cellules permissives sont choisies parmi les hépatocytes primaires humains ou animaux et les cellules du groupe des lignées cellulaires d'hépatocarcinome humain ou animal, telles que les cellules de la lignée d'hépatocarcinome humain HepG2.

18. Procédé selon l'une quelconque des revendications 14 à 17, dans lequel le milieu de culture est le milieu OMEM supplémenté en SVF 10%.

19. Procédé selon l'une quelconque des revendications 14 à 18, selon lequel on met en évidence la présence du virus HCV dans les cellules permissives par RT-PCR et/ou par technique immunologique, telle que par immunofluorescence indirecte, notamment à l'aide d'un anticorps spécifique dudit virus et/ou, par cylométrie de flux.

20. Procédé pour préparer une composition pour la détection dans un échantillon d'anticorps dirigés contre le virus HCV, procédé selon lequel :
on dispose d'un complexe constitué de LVPs associées à des immunoglobulines humaines (LVP/Ig) tel que défini dans l'une quelconque des revendications 1 et 2,
on met en contact ledit complexe avec de la protéine A pour l'obtention d'un complexe LVP/Ig/protéine A,
on met en contact ledit complexe LVP/Ig/protéine A avec au moins un anticorps spécifique d'au moins un récepteur cellulaire pour l'obtention d'un complexe LVP/Ig/protéine A/anticorps
on met en contact, dans un milieu de culture et sous des conditions appropriées, ledit complexe LVP/Ig/protéine A/anticorps et des cellules permissives qui comportent ou expriment à leur surface au moins un récepteur cellulaire ayant la capacité de lier l'anticorps, lesdites cellules permissives étant capables d'assurer la propagation et la réplication du virus HCV *in vitro,* pour obtenir des particules virales de HCV ou des polypeptides, et
on purifie partiellement ou totalement lesdites particules virales de HCV ou lesdits polypeptides.

21. Procédé selon la revendication 20, dans lequel lesdites particules virales ou lesdits polypeptides sont fixés sur un support solide.

22. Procédé pour le chargement in vitro de cellules présentatrices d'antigènes (APCs), selon lequel on dispose d'un complexe constitué de LVPs associées à des immunoglobulines humaines (LVP/Ig) tel que défini dans l'une quelconque des revendications 1 et 2, on met en contact ledit complexe avec de la protéine A pour l'obtention d'un complexe LVP/Ig/protéine A, on met en contact ledit complexe LVP/Ig/protéine A avec au moins un anticorps spécifique d'au moins un récepteur cellulaire des cellules présentatrices d'antigènes (APCs) prélevées chez un être humain ou animal pour l'obtention d'un complexe LVP/Ig/protéine A/anticorps et on met en contact ledit complexe LVP/Ig/protéine A/anticorps spécifique avec lesdites cellules présentatrices d'antigènes.

23. Procédé selon la revendication 22, dans lequel les cellules présentatrices d'antigènes sont les cellules dendritiques et l'anticorps est un anticorps spécifique d'au moins un récepteur cellulaire des cellules dendritiques choisi parmi les « scavenger » récepteur A et B, le récepteur du mannose et les « Toll Like receptors » (TLRs).

24. Procédé pour l'obtention d'anticorps ou de fragments d'anticorps dirigés contre le virus HCV, selon lequel on immunise un animal non-humain avec un complexe LVP/1g/protéine A/anticorps susceptible d'être obtenu par un procédé selon lequel on dispose d'un complexe constitué de LVPs associées à des immunoglobulines humaines (LVP/Ig) tel que défini dans l'une quelconque des revendications 1 et 2, on met en contact ledit complexe avec de la protéine A pour l'obtention d'un complexe LVP/Ig/protéine A, on met en contact ledit complexe LVP/Ig/protéine A avec au moins un anticorps spécifique d'au moins un récepteur cellulaire de cellules présentatrices d'antigènes (APCs) pour l'obtention d'un complexe LVP/Ig/protéine A/anticorps.

25. Procédé selon la revendication 24, dans lequel les cellules présentatrices d'antigènes sont les cellules dendritiques et l'anticorps est un anticorps spécifique d'au moins un récepteur cellulaire des cellules dendritiques choisi parmi les « scavenger » récepteur A et B, le récepteur du mannose et les « Toll Like receptors » (TLRs).

## Claims

1. Complex consisting of lipo-viro-particles (LVPs) associated with human immunoglobulins, having a density of less than 1.063 g/ml, which can be obtained by means of the method according to which:
a sample of a plasma or serum specimen from a patient infected with the hepatitis C virus (HCV) is provided,
the LVPs are separated from said sample by centrifugation as a function of their density, and
the LVPs associated with human immunoglobulins are separated by means of protein A, of anti-human immunoglobulins or of any other molecule capable of binding human immunoglobulins.

2. Complex according to Claim 1, having a density of between 1.0063 and 1.063 g/ml.

3. Method of preparing a complex consisting of LVPs associated with human immunoglobulins and having a density of less than 1.063 g/ml, preferably of between 1.0063 and 1.063 g/ml, according to which:
a sample of a plasma or serum specimen from a patient infected with HCV is provided,
the LVPs are separated from said sample by centrifugation as a function of their density, and
the LVPs associated with human immunoglobulins are separated by means of protein A, of anti-human immunoglobulins or of any other molecule capable of binding human immunoglobulins.

4. Method according to Claim 3, in which the protein A, the anti-human immunoglobulins or other molecule capable of binding human immunoglobulins are coupled to a support, such as beads or sepharose.

5. Method of culturing the HCV virus, *in vitro,* according to which a complex consisting of LVPs associated with human immunoglobulins according to Claims 1 and 2, optionally prepared according to Claims 3 and 4, is brought into contact, in a culture medium and under conditions that are appropriate, with permissive cells that comprise at their surface at least one type of immunoglobulin Fc fragment receptor, said permissive cells being capable of ensuring the propagation and the replication of the HCV virus *in vitro.*

6. Method according to Claim 5, according to which the permissive cells are chosen from the group consisting of mononuclear cells, such as bone marrow-derived stem cells, monoblasts, promonocytes, monocytes and macrophages, macrophage precursor cells, B lymphocytes, NK cells, human or animal primary hepatocytes, cells of the human or animal hepatocarcinoma cell line group, Kupffer cells, dendritic cells, epithelial cells, vascular endothelial cells, mast cells, Langerhans cells, syncytiotrophoblasts, eosinophil, basophil and neutrophil polymorphonuclear cells, and platelets.

7. Method according to Claim 6, in which the macrophages are preferably chosen from the group which consists of histiocytes, alveolar macrophages, spleen and lymphoid tissue macrophages, Kupffer cells, osteoclasts, synovial type A cells, tissue macrophages and the precursor cells from which these cells are derived.

8. Method according to Claim 6, in which the permissive cells are primary or animal hepatocytes, cells of the human or animal hepatocarcinoma cell line group or Kupffer cells which have at least one type of immunoglobulin Fc fragment receptor and/or at least one lipoprotein receptor such as the LDL-receptor and the lipolysis-stimulated receptor.

9. Method according to any one of Claims 5 to 8, in which the culture medium is RPMI 1640 medium supplemented with 1% penicillin, 1% streptomycin, 2 mM glutamine and 10% SVF, and also optionally comprising 50 *µ*M beta-mercaptoethanol.

10. Method according to any one of Claims 6 to 9, in which the presence of the HCV virus in the permissive cells is demonstrated by RT-PCR and/or by an immunological technique, such as by indirect immunofluorescence, in particular using an antibody specific for said virus, and/or by flow cytometry.

11. Method for preparing a composition for detecting, in a specimen, antibodies directed against the HCV virus, according to which method:
a complex consisting of LVPs associated with human immunoglobulins according to Claims 1 and 2, optionally prepared according to Claims 3 and 4, is brought into contact, in a culture medium and under conditions that are appropriate, with permissive cells which comprise at their surface at least one type of immunoglobulin Fc fragment receptor, said permissive cells being capable of ensuring the propagation and the replication of the HCV virus in vitro, so as to obtain HCV viral particles or polypeptides, and
said HCV viral particles or said polypeptides are partially or completely purified.

12. Method according to Claim 11, in which said viral particles or said polypeptides are attached to a solid support.

13. Method for obtaining antibodies or antibody fragments directed against the HCV virus, according to which a non-human animal is immunized with a complex according to Claim 1 or 2, optionally prepared by means of a method according to Claim 3 or 4.

14. Method of culturing the HVC virus, in vitro, according to which a complex consisting of LVPs associated with human immunoglobulins (LVP/Ig) as defined in either one of Claims 1 and 2 is provided, said complex is brought into contact with protein A so as to obtain an LVP/Ig/protein A complex, said LVP/Ig/protein A complex is brought into contact with at least one antibody specific for at least one cellular receptor so as to obtain an LVP/Ig/protein A/antibody complex, and said LVP/Ig/protein A/antibody complex is brought into contact, in a culture medium and under conditions that are appropriate, with permissive cells which comprise or express at their surface at least one cellular receptor having the ability to bind the antibody; said permissive cells being capable of ensuring the propagation and the replication of the HCV virus in vitro.

15. Method according to Claim 14, in which the protein A is coupled to a support, such as beads or sepharose.

16. Method according to Claim 14 or 15, in which the antibody is an antibody specific for at least one of the LDL receptors and the permissive cells comprise or express at their surface at least one LDL receptor.

17. Method according to Claim 16, in which the permissive cells are chosen from human or animal primary hepatocytes and cells of the human or animal hepatocarcinoma cell line group, such as cells of the HepG2 human hepatocarcinoma line.

18. Method according to any one of Claims 14 to 17, in which the culture medium is DMEM medium supplemented with 10% SVF.

19. Method according to any one of Claims 14 to 18, according to which the presence of the HCV virus in the permissive cells is demonstrated by RT-PCR and/or by an immunological technique, such as by indirect immunofluorescence, in particular using an antibody specific for said virus, and/or by flow cytometry.

20. Method for preparing a composition for detecting, in a specimen, antibodies directed against the HCV virus, according to which method:
a complex consisting of LVPs associated with human immunoglobulins (LVP/Ig) as defined in either one of Claims 1 and 2 is provided,
said complex is brought into contact with protein A so as to obtain an LVP/Ig/protein A complex,
said LVP/Ig/protein A complex is brought into contact with at least one antibody specific for at least one cellular receptor so as to obtain an LVP/Ig/protein A/antibody complex,
said LVP/Ig/protein A/antibody complex is brought into contact, in a culture medium and under conditions that are appropriate, with permissive cells which comprise or express at their surface at least one cellular receptor having the ability to bind the antibody, said permissive cells being capable of ensuring the propagation and the replication of the HCV virus *in vitro,* so as to obtain HCV viral particles or polypeptides, and
said HCV viral particles or said polypeptides are partially or completely purified.

21. Method according to Claim 20, in which said viral particles or said polypeptides are attached to a solid support.

22. Method for loading antigen-presenting cells (APCs), *in vitro,* according to which a complex consisting of LVPs associated with human immunoglobulins (LVP/Ig) as defined in either one of Claims 1 and 2 is provided, said complex is brought into contact with protein A so as to obtain an LVP/Ig/protein A complex, said LVP/Ig/protein A complex is brought into contact with at least one antibody specific for at least one cellular receptor of the antigen-presenting cells (APCs) taken from a human being or animal so as to obtain an LVP/Ig/protein A/antibody complex, and said LVP/Ig/protein A/specific antibody complex is brought into contact with said antigen-presenting cell.

23. Method according to Claim 22, in which the antigen-presenting cells are dendritic cells and the antibody is an antibody specific for at least one cellular receptor of dendritic cells, chosen from scavenger receptors A and B, the mannose receptor and Toll Like receptors (TLRs).

24. Method for obtaining antibodies or antibody fragments directed against the HCV virus, according to which a non-human animal is immunized with an LVP/Ig/protein A/antibody complex that can be obtained by means of a method according to which a complex consisting of LVPs associated with human immunoglobulins (LVP/Ig) as defined in either one of Claims 1 and 2 is provided, said complex is brought into contact with protein A so as to obtain an LVP/Ig/protein A complex, and said LVP/Ig/protein A complex is brought into contact with at least one antibody specific for at least one cellular receptor of antigen-presenting cells (APCs) so as to obtain an LVP/Ig/protein A/antibody complex.

25. Method according to Claim 24, in which the antigen-presenting cells are dendritic cells and the antibody is an antibody specific for at least one cellular receptor of dendritic cells, chosen from scavenger receptors A and B, the mannose receptor and Toll Like receptors (TLRs).

## Patentansprüche

1. Komplex bestehend aus Lipo-Virus-Partikeln (LVPs) in Kombination mit Human-Immunglobulinen, der eine Dichte unterhalb 1,063 g/ml aufweist und durch ein Verfahren gewonnen werden kann, bei dem man:
über eine Probe eines Plasma- oder Serummaterials eines mit Hepatitis-C-Virus (HCV) infizierten Patienten verfügt,
die LVPs von dieser Probe mittels Zentrifugation aufgrund ihrer Dichte abtrennt, und
die mit Human-Immunglobulinen assoziierten LVPs mit Hilfe von Protein A, Humman-anti-Immunglobulinen oder beliebigen anderen Molekülen, die fähig sind, an die Human-Immunglobuline zu binden, abtrennt.

2. Komplex nach Anspruch 1 mit einer Dichte zwischen 1,0063 und 1,063 g/ml.

3. Verfahren zur Herstellung eines Komplexes bestehend aus LVPs in Kombination mit Human-Immunglobulinen und mit einer Dichte unterhalb 1,063 g/ml, vorzugsweise zwischen 1,0063 und 1,063 g/ml, bei dem man:
über eine Probe eines Plasma- oder Serummaterials eines mit Hepatitis-C-Virus (HCV) infizierten Patienten verfügt,
die LVPs von dieser Probe mittels Zentrifugation aufgrund ihrer Dichte abtrennt, und
die mit Human-Immunglobulinen assoziierten LVPs mit Hilfe von Protein A, Humman-anti-Immunglobulinen oder beliebigen anderen Molekülen, die fähig sind, an die Human-Immunglobuline zu binden, abtrennt.

4. Verfahren nach Anspruch 3, bei dem das Protein A, die Antikörper gegen Human-Immunglobuline oder andere Moleküle, die fähig sind, an Human-Immunglobuline zu binden, an einen Träger wie Perlon oder Sepharose gekoppelt sind.

5. Verfahren zur in vitro-Kultivierung von HCV-Virus, bei dem man einen Komplex bestehend aus LVPs in Kombination mit Human-Immunglobulinen nach Anspruch 1 und 2, der gegebenenfalls nach Anspruch 3 und 4 hergestellt worden ist, und permissive Zellen, die an ihrer Oberfläche mindestens eine Art von Rezeptor für das Fragment Fc der Immunglobuline aufweisen, in einem Kulturmedium unter geeigneten Bedingungen in Kontakt bringt, wobei die permissiven Zellen fähig sind, die Vermehrung und Replikation des HCV-Virus in vitro zu gewährleisten.

6. Verfahren nach Anspruch 5, bei dem die permissiven Zellen aus der Gruppe mononukleäre Zellen wie Knochenmarkstammzellen, Monoplasten, Promonocyten Monocyten und Makrophagen, Makrophagen-Vorläuferzellen, B-Lymphocyten, NK-Zellen, primäre humane oder tierische Hepatocyten, Zellen der Gruppe der tierischen oder menschlichen Leberkarzinomzellinien, Kuppfer-Zellen, Dendritenzellen, Epitelzellen, Gefäßendothelzellen, Mastocyten, Langerhanssche Zellen, Syncytiotrophoblasten, eosinophile, basophile und neutralophile polynukleäre Zellen sowie Blutplättchen stammen.

7. Verfahren nach Anspruch 6, wobei die Makrophagen vorzugsweise aus der Gruppe Histiocyten, alveoläre Makrophagen, Ratten-Makrophagen, Lymphoidgewebe-Makrophagen, Kuppfer-Zellen, Osteoklasten, Typ-A-Synovialzellen, Gewebemakrophagen und Vorläuferzellen, von denen sich diese Zellen ableiten, stammen.

8. Verfahren nach Anspruch 6, bei dem es sich bei den permissiven Zellen um primäre oder tierische Hepatocyten, Zellen der Gruppe der menschlichen oder tierischen Leberkarzinomzellinien oder Kuppfer-Zellen, die mindestens eine Art von Rezeptor für das Fc-Fragment der Immunglobuline und/oder mindestens einen Rezeptor für Lipoproteine wie den LDL-Rezeptor und den Lipolysis Stimulated Receptor aufweisen, handelt.

9. Verfahren nach einem der Ansprüche 5 bis 8, wobei es sich bei dem Kulturmedium um das RPMI-1640-Medium mit Zusatz von 1% Penicillin, Streptomycin, 2 mM Glutamin, 10% SVF, das weiterhin gegebenenfalls 50 µM beta-Mercaptoethanol enthält, handelt.

10. Verfahren nach einem der Ansprüche 6 bis 9, bei dem man das Vorhandensein des HCV-Virus in den permissiven Zellen mittels RT-PCR und/oder immunologisch, wie mittels indirekter Immunfluoreszenz insbesondere mit Hilfe eines spezifischen Antikörpers dieses Virus, und/oder mittels Durchflußcytometrie nachweist.

11. Verfahren zur Herstellung einer Zusammensetzung für den Nachweis von gegen das HCV-Virus gerichteten Antikörpern in einer Probe, bei dem man:
einen Komplex bestehend aus LVPs in Kombination mit Human-Immunglobulinen nach Anspruch 1 und 2, der gegebenenfalls nach Anspruch 3 und 4 hergestellt worden ist, und permissive Zellen, die an ihrer Oberfläche mindestens eine Art von Rezeptor für das Fc-Fragment der Immunglobuline aufweisen, in einem Kulturmedium unter geeigneten Bedingungen in Kontakt bringt, wobei die permissiven Zellen fähig sind, die Vermehrung und Replikation des HCV-Virus in vitro zu gewährleisten, wodurch man HCV-Virus-Partikel oder Polypeptide erhält, und
diese HCV-Viruspartikel oder diese Polypeptide teilweise oder vollständig aufreinigt.

12. Verfahren nach Anspruch 11, bei dem die Viruspartikel oder Polypeptide auf einem festen Träger fixiert sind.

13. Verfahren für die Gewinnung von Antikörpern oder Antikörperfragmenten, die gegen das HCV-Virus gerichtet sind, bei dem man ein nichtmenschliches Tier mit einem Komplex nach Anspruch 1 oder 2, der gegebenenfalls nach einem Verfahren nach Anspruch 3 oder 4 hergestellt worden ist, immunisiert.

14. Verfahren für die in-vitro-Kultivierung des HCV-Virus, bei dem man über einen Komplex bestehend aus LVPs in Kombination mit Human-Immunglobulinen (LVP/Ig) wie in einem der Ansprüche 1 und 2 definiert verfügt, diesen Komplex mit Protein A in Kontakt bringt, wodurch man einen LVP/Ig/Protein-A-Komplex erhält, diesen LVP/Ig/Protein-A-Komplex mit mindestens einem Antikörper der für mindestens einen Zellrezeptor spezifisch ist, in Kontakt bringt, wodurch man einen LVP/Ig/Protein-A/Antikörper-Komplex erhält, und man diesen LVP/Ig/Protein-A/Antikörper-Komplex und permissive Zellen, die an ihrer Oberfläche mindestens einen Zellrezeptor mit der Fähigkeit, den Antikörper zu binden, aufweisen oder exprimieren, in einem Kulturmedium unter geeigneten Bedingungen in Kontakt bringt; wobei die permissiven Zellen fähig sind, die Vermehrung und Replikation des HCV-Virus in vitro zu gewährleisten.

15. Verfahren nach Anspruch 14, bei dem das Protein A an einen Träger wie Perlon oder Sepharose gekoppelt ist.

16. Verfahren nach Anspruch 14 oder 15, wobei der Antikörper für mindestens einen der LDL-Rezeptoren spezifisch ist und die permissiven Zellen an ihrer Oberfläche mindestens einen LDL-Rezeptor aufweisen oder exprimieren.

17. Verfahren nach Anspruch 16, wobei die permissiven Zellen aus der Gruppe der menschlichen oder tierischen primären Hepatocyten und den Zellen aus der Gruppe der menschlichen oder tierischen Leberkarzinomzellinien, wie Zellen der menschlichen Leberkarzinomlinien HepG2, stammen.

18. Verfahren nach einem der Ansprüche 14 bis 17, wobei es sich bei dem Kulturmedium um das DMEM-Medium mit einem Zusatz von 10% SVF handelt.

19. Verfahren nach einem der Ansprüche 14 bis 18, bei dem man das Vorhandensein des HCV-Virus in den permissiven Zellinien mittels RT-PCR und/oder immunologisch wie mittels indirekter Immunfluoreszenz, insbesondere mit Hilfe eines für dieses Virus spezifischen Antikörpers, und/oder Durchflußcytometrie nachweist.

20. Verfahren zur Herstellung einer Zusammensetzung für den Nachweis von gegen das HCV-Virus gerichteten Antikörpern in einer Probe, bei dem man:
über einen Komplex bestehend aus LVPs in Kombination mit Human-Immunglobulinen (LVP/Ig) wie in einem der Ansprüche 1 und 2 definiert verfügt, diesen Komplex mit Protein A in Kontakt bringt, wodurch man einen LVP/Ig/Protein-A-Komplex erhält, diesen LVP/Ig/Protein-A-Komplex mit mindestens einem Antikörper der für mindestens einen Zellrezeptor spezifisch ist, in Kontakt bringt, wodurch man einen LVP/Ig/Protein-A/Antikörper-Komplex erhält, und man diesen LVP/Ig/Protein-A/Antikörper-Komplex und permissive Zellen, die an ihrer Oberfläche mindestens einen Zellrezeptor mit der Fähigkeit, den Antikörper zu binden, aufweisen oder exprimieren, in einem Kulturmedium unter geeigneten Bedingungen in Kontakt bringt, wobei die permissiven Zellen, fähig sind, die Vermehrung und Replikation des HCV-Virus in vitro zu gewährleisten, wodurch man zu HCV-Viruspartikeln oder Polypeptiden gelangt, und
man die HCV-Virsupartikel oder die Polypeptide teilweise oder vollständig aufreinigt.

21. Verfahren nach Anspruch 20, bei dem die Viruspartikel oder Polypeptide auf einem festen Träger fixiert sind.

22. Verfahren zur *in-vitro*-Beladung von antigenpräsentierenden Zellen (APCs), bei dem man über einen Komplex bestehend aus LVPs in Kombination mit Human-Immunglobulinen (LVP/Ig) wie in einem der Ansprüche 1 und 2 definiert verfügt, diesen Komplex mit Protein A in Kontakt bringt, wodurch man einen LVP/Ig/Protein-A-Komplex erhält, diesen LVP/Ig/Protein-A-Komplex mit mindestens einem Antikörper, der für mindestens einen Zellrezeptor von antigenpräsentierenden Zellen (APCs), die von einem Menschen oder Tier entnommen wurden, spezifisch ist, in Kontakt bringt, wodurch man zu einem LVP/Ig/Protein-A/Antikörper-Komplex gelangt, und man diesen LVP/Ig/Protein-A/Antikörper-Komplex mit den antigenpräsentierenden Zellen in Kontakt bringt.

23. Verfahren nach Anspruch 22, wobei es sich bei den antigenpräsentierenden Zellen um Dendritenzellen handelt und bei dem Antikörper um einen Antikörper, der für mindestens einen Zellrezeptor von Dendritenzellen spezifisch ist, aus der Gruppe "scavenger"-Rezeptor A und B, Manoserezeptor und "Toll-Like-Receptors" (TLRs) handelt.

24. Verfahren zur Gewinnung von Antikörpern oder Antikörperfragmenten, die gegen das HCV-Virus gerichtet sind, bei dem man ein nichtmenschliches Tier mit einem LVP/Ig/Protein-A/Antikörper-Komplex immunisiert, der durch ein Verfahren gewonnen werden kann, bei dem man über einen Komplex bestehend aus LVPs in Kombination mit Human-Immunglobulinen (LVP/Ig) wie in einem der Ansprüche 1 und 2 definiert verfügt, diesen Komplex mit Protein A in Kontakt bringt, wodurch man einen LVP/Ig/Protein-A-Komplex erhält, diesen LVP/Ig/Protein-A-Komplex mit mindestens einem Antikörper der für mindestens einen Zellrezeptor von antigenpräsentierenden Zellen (APCs), spezifisch ist, in Kontakt bringt, wodurch man zu einem LVP/Ig/Protein-A/Antikörper-Komplex gelangt.

25. Verfahren nach Anspruch 24, wobei es sich bei den antigenpräsentierenden Zellen um Dendritenzellen handelt und bei dem Antikörper um einen Antikörper, der für mindestens einen Zellrezeptor von Dendritenzellen spezifisch ist, aus der Gruppe "scavenger"-Rezeptor A und B, Manoserezeptor und "Toll-Like-Receptors" (TLRs) handelt.
